# EUROPÄISCHE PATENTSCHRIFT

(11) **EP 3 601 510 B1**
(45) Veröffentlichungstag und Bekanntmachung des Hinweises auf die Patenterteilung: **30.04.2025**
(21) Anmeldenummer: 17809248.2
(22) Anmeldetag: 27.11.2017
(51) Int. Cl.: C11B 9/00, A23L 27/20

(54) **2,3,6-TRIMETHYLCYCLOHEXANOL ALS RIECH- UND/ODER AROMASTOFF**
2,3,6-TRIMETHYLCYCLOHEXANOL AS A SCENTING AND/OR FLAVORING AGENT
2,3,6-TRIMÉTHYLCYCLOHEXANOL UTILISÉ COMME PARFUM ET/OU ARÔME

(30) Priorität: 21.03.2017 WO PCT/EP2017/056708
(43) Veröffentlichungstag der Anmeldung: 05.02.2020
(73) Patentinhaber: Symrise AG, 37603 Holzminden (DE)
(72) Erfinder: HÖLSCHER, Bernd, 31785 Hameln (DE); MEIER, Manfred, 37699 Fürstenberg (DE)
(74) Vertreter: Eisenführ Speiser
(86) Internationale Anmeldenummer: PCT/EP2017/080536
(87) Internationale Veröffentlichungsnummer: WO 2018/171917

(56) Entgegenhaltungen:
- EP-A1- 2 389 922
- DE-A1- 2 317 000
- DE-A1- 3 003 518
- JP-B1- S5 022 548
- DATABASE CAPLUS [online] CHEMICAL ABSTRACTS SERVICE, COLUMBUS, OHIO, US; "Alkylcyclohexanols", XP002772579, Database accession no. 1976:58762

## Beschreibung

Die vorliegende Erfindung betrifft primär die Verwendung von 2,3,6-Trimethylcyclohexanol, d.h. einer Verbindung der Formel (I) wie hierin beschrieben, als Riech- und/oder Aromastoff zum Vermitteln, Modifizieren und/oder Verstärken einer oder mehrerer Geruchs- und/oder Geschmacksnoten wie hierin beschrieben. Die Erfindung betrifft zudem neue Riech- und/oder Aromastoffkompositionen umfassend die Verbindung der Formel (I) wie hierin beschrieben, parfümierte und/oder aromatisierte Artikel umfassend die Verbindung der Formel (I) wie hierin beschrieben sowie diverse Verfahren zum Vermitteln, Modifizieren und/oder Verstärken bestimmter Geruchs- und/oder Geschmacksnoten.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

Trotz einer Vielzahl bereits vorhandener Riech- und/oder Aromastoffe besteht in der Parfüm- und Aromenindustrie auch weiterhin ein genereller Bedarf an neuen Riech- und/oder Aromastoffen. So besteht beispielsweise ein Bedarf an Riech- und/oder Aromastoffen, die in der Lage sind (in Riech- und/oder Aromastoffkompositionen) neben einer primären Duft-/Geschmacksnote weitere interessante Noten zu erzeugen und mit ihren neuartigen bzw. originellen olfaktorischen Eigenschaften die Möglichkeiten des Parfümeurs bzw. Flavoristen zu erweitern. Insbesondere besteht ein Interesse an Riechstoffen mit Duftnoten, welche in der Lage sind, eine harmonische Kombination mit blumigen und/oder fruchtigen Riechstoffen einzugehen. Vorzugsweise sollte dabei eine Überlagerung der unterschiedlichen geruchlichen Aspekte und Noten erfolgen, um dadurch einen insgesamt komplexen Geruchseindruck zu erzeugen.

Für die Kreation neuartiger Kompositionen besteht ständiger Bedarf an Riech- und/oder Aromastoffen mit besonderen sensorischen Eigenschaften, die geeignet sind, als Grundlage für die Komposition von neuartigen Parfüms oder Aromen mit komplexem sensorischen Charakter zu dienen. Bevorzugte gesuchte Riech- und/oder Aromastoffe sollten neben einer bestimmten Note weitere Noten und Aspekte aufweisen, die ihnen Charakter und Komplexität verleihen.

Die Suche nach geeigneten Stoffen, die zur vorliegenden Erfindung führte, wurde durch folgende Sachverhalte erschwert:
- die Mechanismen der Geruchswahrnehmung sind nicht ausreichend bekannt;
- die Zusammenhänge zwischen der speziellen Geruchswahrnehmung einerseits und der chemischen Struktur des zugehörigen Riechstoffs andererseits sind nicht hinreichend erforscht;
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Die primäre Aufgabe bestand nun darin, Riech- und/oder Aromastoffe zu finden, die ein interessantes, vorzugsweise komplexes, und originelles sensorisches Profil aufweisen und sich als Riechstoffe für den Einsatz in z.B. der Parfümerie bzw. als Aromastoffe zur Aromatisierung von z.B. verzehrbaren Zubereitungen eignen.

Es wurden im Rahmen der vorliegenden Erfindung insbesondere Stoffe gesucht, die eine, mehrere oder sämtliche der Noten grün, holzig, animalisch, erdig, ambrierend, fruchtig, blumig und Leder-Note aufweisen bzw. vermitteln, modifizieren und/oder verstärken können.

Die gesuchten Stoffe sollten die Herstellung von neuartigen Riech- und/oder Aromastoffkompositionen mit besonderen geruchlichen bzw. geschmacklichen Noten und Aspekten ermöglichen. Von Vorteil wären dabei solche Stoffe, welche insbesondere zur Kombination mit weiteren Riech- oder Aromastoffen geeignet sind, die eine holzige, animalische, erdige und ambrierende Note aufweisen.

Daneben sollten die diese primäre Aufgabe erfüllenden Riech- und/oder Aromastoffe vorzugsweise über ihre primären, nämlich geruchlichen bzw. geschmacklichen, Eigenschaften hinaus zusätzliche positive Sekundäreigenschaften besitzen, wie z.B. eine hohe Stabilität unter bestimmten Anwendungsbedingungen, eine hohe Ausgiebigkeit, ein gutes Haftungsvermögen, eine hohe Substantivität oder geruchs- und/oder geschmacksverstärkende Eigenschaften (sogenannte Booster- oder Enhancer-Wirkung), und/oder in Kombination mit anderen Riech- und/oder Aromastoffen deren Natürlichkeit, Frische, Fülle, (Strahl-)Kraft und/oder Ausstrahlung abrunden, so dass sensorisch bemerkenswerte Effekte erzielt werden können.

DE 2317000 von Wilkinson Sword Ltd. betrifft einnehmbare, örtlich aufbringbare und andere Zusammensetzungen mit einem physiologischen Kühleffekt auf der Haut und auf den Schleimhäuten des Körpers, besonders den Schleimhäuten der Nase und der Atemwege. 2,3,6-Trimethylcyclohexanol wird von DE 2317000 nicht offenbart.

DE 3003518 betrifft die Verwendung von 1-Äthynyl-2,2,6-trimethyl-cyclohexanol als Parfüm- und Aromabestandteil.

JP S50 22548B offenbart ein Verfahren zur Herstellung von 2,3,6-Trimethylcyclohexanol ausgehend von 2,3,6-Trimethylphenol. Die geruchlichen Eigenschaften der eingesetzten oder erhaltenen Stoffe bzw. Mischungen werden nicht erwähnt.

Erfindungsgemäß gelöst wird die primäre gestellte Aufgabe durch die Verwendung der Verbindung der Formel (I) (2,3,6-Trimethylcyclohexanol, CAS-Nr. 58210-03-0) 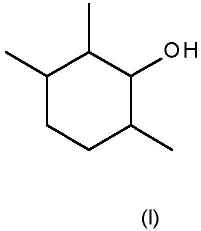 als Riech- und/oder Aromastoff zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise einer oder mehrerer der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note.

Die erfindungsgemäß einzusetzende Verbindung der Formel (I) kann als beliebiges Stereoisomer vorliegen und damit auch in beliebiger optisch aktiver Form. Im Rahmen der vorliegenden Erfindung können auch beliebige Mischungen von Stereoisomeren der Verbindung der Formel (I) verwendet werden, beispielsweise ein Diastereomerengemisch, Enantiomerengemisch oder ein Racemat, d.h. unter den Begriff "Verbindung der Formel (I)" fallen im Rahmen dieses Textes auch die besagten beliebigen Mischungen verschiedener Stereoisomere der Verbindung der Formel (I).

Das hierin für eine Verbindung der Formel (I) Gesagte, insbesondere die hierin beschrieben Vorteile, gelten auch für eine erfindungsgemäß zu verwendende bzw. einzusetzende Mischung von Stereoisomeren der Verbindung der Formel (I) (s. oben).

Die Verbindung der Formel (I) verfügt über ganz eigenständige olfaktorische Eigenschaften, die sich deutlich von bekannten Riech- und/oder Aromastoffen abheben und diese auch übertreffen. Die Eignung der Verbindung der Formel (I) als Riech- und/oder Aromastoff war bislang nicht bekannt. Es ist daher besonders überraschend, dass auf dem bereits gut untersuchten Gebiet ein Riech- und/oder Aromastoff mit wertvollen, interessanten und komplexen Geruchseigenschaften gefunden werden konnte.

Vorzugsweise betrifft die erfindungsgemäße Verwendung eine Verwendung zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch und Leder-Note, vorzugsweise einer oder mehrerer der Geruchs- und/oder Geschmacksnoten ausgewählt aus der Gruppe bestehend aus den Noten grün, holzig, animalisch, erdig, ambrierend, fruchtig, blumig und Leder-Note.

Eine bevorzugte Ausführungsform betrifft die Verwendung der Verbindung der Formel (I) zum Vermitteln einer, zweier oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch.

Eine weitere bevorzugte Ausführungsform betrifft die Verwendung der Verbindung der Formel (I) zum Vermitteln einer grünen und/oder fruchtigen, vorzugsweise einer grünen und fruchtigen, vorzugsweise einer ananasartigen, Geruchs- und/oder Geschmacksnote und zusätzlich einer, zweier oder mehrerer Geruchs- und/oder Geschmacksnoten ausgewählt aus der Gruppe bestehend aus den Noten krautig, frisch, blumig, holzig, süß, erdig, fettig, metallisch und balsamisch.

Die Tatsache, dass die erfindungsgemäß einzusetzende Verbindung der Formel (I) einen sehr komplexen und vielfältigen Geruchs- und Geschmackseindruck zu vermitteln mag, der sonst meist nur von Mischungen mehrerer Komponenten (wie beispielsweise ätherischen Ölen oder Gewürzmischungen) erreicht werden kann, ist besonders überraschend.

Über die primären, nämlich geruchlichen und geschmacklichen, Eigenschaften hinaus verfügt die Verbindung der Formel (I) zusätzlich über positive Sekundäreigenschaften, insbesondere ein gutes Haftungsvermögen und eine hohe Substantivität im Vergleich zu Riechstoffen mit ähnlichen Geruchseigenschaften, sowie eine hohe Stabilität in bestimmten Medien und Zubereitungen und eine hohe Ausgiebigkeit und ist zudem biologisch abbaubar.

Die erfindungsgemäß einzusetzende Verbindung der Formel (I) weist insbesondere in weitgehend neutralen, insbesondere jedoch in alkalischen und/oder in oxidierenden Medien eine hohe, ganz hervorragende Stabilität auf. Insbesondere wegen dieser Eigenschaften eignet sich die Verbindung der Formel (I) ausgezeichnet für die Verwendung als Riech- und/oder Aromastoff, und zwar insbesondere, wenn sie in parfümierten oder aromatisierten Artikeln (Zubereitungen) eingesetzt wird, die einen pH-Wert von 5,5 oder größer besitzen, vorzugsweise von größer oder gleich 6, bevorzugt von größer 7, weiter bevorzugt von größer 7,5, insbesondere bevorzugt von größer 8; ferner in oxidierend wirkenden Zubereitungen, die vorzugsweise einen pH-Wert von größer oder gleich 7 aufweisen, bevorzugt in oxidierend wirkenden Zubereitungen mit einem pH-Wert von größer oder gleich 8. Die angegebenen pH-Werte beziehen sich dabei jeweils auf bei 25°C gemessene Werte.

Die erfindungsgemäß zu verwendende Verbindung der Formel (I) kann vorteilhafterweise die Intensität einer Riech- bzw. Aromastoffmischung (Riech- und/oder Aromastoffkomposition) verstärken und das Gesamtbild der Mischung geruchlich und/oder geschmacklich abrunden und kann eingesetzt werden, um einer Riech- und/oder Aromastoffkomposition mehr Fülle, Frische, (Strahl-)Kraft, Ausstrahlung, Glanz, Abrundung, Harmonie und/oder Natürlichkeit zu verleihen.

Weiterhin eignet sich die Verbindung der Formel (I) als Mittel zum Erhöhen der Substantivität und/oder Retention einer Riech- und/oder Aromastoffmischung und/oder als Fixateur.

Sofern in Rahmen dieses Textes versehentlich eine Unstimmigkeit zwischen dem chemischen Namen und der dargestellten Strukturformel der Verbindung der Formeln (I) auftreten sollte, gilt die dargestellte Strukturformel.

Vorteilhafterweise zeigt die erfindungsgemäß einzusetzende Verbindung der Formel (I) auch sensorische Eigenschaften der Ocimen-Art und weist eine Chlor-Skatol-Note auf.

Durch die Chlor-Skatol-Note ist die Verbindung der Formel (I) u. a. als Signalstoff bei antibakteriellen Anwendungen geeignet.

Eine weitere bevorzugte Ausführungsform betrifft daher die Verwendung der Verbindung der Formel (I) zum Vermitteln, Modifizieren und/oder Verstärken der Geruchs- und/oder Geschmacksnote Chlor.

In vielen Ländern wird eine chlorartige Geruchs- oder Geschmacksnote, insbesondere Geruchsnote, mit dem Eindruck von Sauberkeit, Hygiene und antibakteriellen Eigenschaften in Verbindung gebracht. Die erfindungsgemäße Verwendung ist aus diesem Grund besonders vorteilhaft, da durch sie eine Chlor-Note vermittelt, modifiziert und/oder verstärkt werden kann, ohne dass den erfindungsgemäßen, die Verbindung der Formel (I) enthaltenden, Riech- und/oder Aromastoffkompositionen bzw. parfümierten und/oder aromatisierten Artikeln (wie weiter unten beschrieben) tatsächlich Chlor zugesetzt werden muss bzw. diese vorteilhafter einen wesentlich geringeren tatsächlichen Chlor-Gehalt aufweisen können, der ohne die geruchliche Verstärkung durch die Verbindung der Formel (I) unter Umständen überhaupt nicht sensorisch detektierbar wäre. Diese Chlor-Signalwirkung der Verbindung der Formel (I), d.h. die Vermittlung des Eindrucks, dass eine erfindungsgemäße Riech- und/oder Aromastoffkomposition bzw. ein erfindungsgemäßer parfümierter und/oder aromatisierter Artikel (wie weiter unten beschrieben) aufgrund ihrer / seiner sensorisch wahrnehmbaren Chlor-Note besonders sauber, hygienisch und/oder antibakteriell ist bzw. wirkt, ist bei vielen Konsumenten besonders beliebt und insbesondere vorteilhaft einsetzbar in Mundpflegemitteln, wie beispielsweise Mundwässern und Zahnpflegemitteln, und Oberflächenreinigern, wie beispielsweise WC-Reinigern und Allzweckreinigern.

Die Verbindung der Formel (I) ist aus der Literatur bereits bekannt (siehe z.B. WO2010122178, WO2014198602, WO2010097479, EP2389922, EP50229 und US20110294876). Allerdings werden im Stand der Technik deren sensorische Eigenschaften und deren Eignung als Riech- und/oder Aromastoff nicht beschrieben.

Die Herstellung der erfindungsgemäß zu verwendenden Verbindung der Formel (I) kann mittels an sich bekannter Reaktionen und Verfahren erfolgen. Zum Beispiel kann das entsprechende Phenol (CAS-Nr. 2416-94-6) oder ein entsprechendes Keton (z.B.: CAS-Nr. 20030-30-2) zu der Verbindung der Formel (I), mit entsprechenden Katalysatoren und den entsprechenden Bedingungen, hydriert werden (die dafür eingesetzten Edukte sind kommerziell erhältlich):

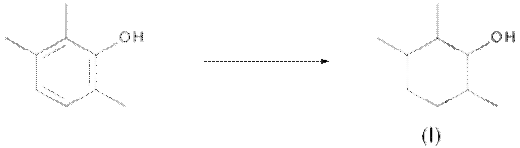

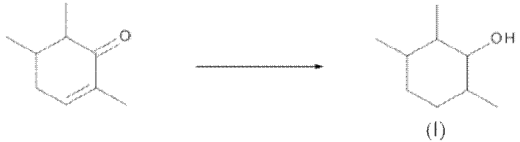

Herstellung der Verbindung der Formel (I) aus dem entsprechenden Phenol (oben) bzw. einem entsprechenden Keton (unten) durch Hydrierung

Die erfindungsgemäß einzusetzende Verbindung der Formel (I) wird im Rahmen der erfindungsgemäßen Verwendung üblicherweise in einer sensorisch wirksamen Menge eingesetzt, d.h. in einer Gesamtmenge, in der sie eine sensorische Wirkung entfaltet. Vorzugsweise wird die erfindungsgemäß einzusetzende Verbindung der Formel (I) zusammen mit anderen Riech- und/oder Aromastoffen eingesetzt. Solche Riech- und/oder Aromastoffkompositionen können in üblicher Weise hergestellt werden, beispielsweise durch einfaches Vermischen oder Homogenisieren der Bestandteile. Bei diesen weiteren Riech- uns/oder Aromastoffen kann es sich um beliebige weitere Riech- und/oder Aromastoffe (bevorzugte Kombinationen ergeben sich aus den nachfolgenden Ausführungen) handeln.

Ein weiterer Aspekt der vorliegenden Erfindung betrifft daher eine Riech- und/oder Aromastoffkomposition umfassend oder bestehend aus der Verbindung der Formel (I) 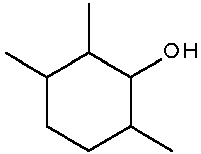 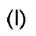 und einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt, und wobei die Gesamtmenge an Verbindung der Formel (I) in der Riech- und/oder Aromastoffkomposition in einer Menge enthalten ist, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens eine der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, zu vermitteln und/oder zu verstärken und/oder eine oder mehrere Geruchs- und/oder Geschmacksnoten eines bzw. des / der weiteren Riech- und/oder Aromastoffs/e der Riech- und/oder Aromastoffkomposition in Richtung einer oder mehrerer dieser Geruchs- und/oder Geschmacksnoten zu modifizieren.

Hierbei ist es bevorzugt, wenn die Gesamtmenge an Verbindung der Formel (I), bezogen auf das Gesamtgewicht der Riech- und/oder Aromastoffkomposition, im Bereich von 0,0001 bis 90 Gew.-%, vorzugsweise 0,05 bis 80 Gew.-%, 0,1 bis 70 Gew.-%, 0,25 bis 50 Gew.-%, 0,5 bis 40 Gew.-% oder 0,75 bis 25 Gew.-%, liegt.

Sofern die Verbindung der Formel (I) hauptsächlich eingesetzt wird, um einer Riech- und/oder Aromastoffkomposition mehr Frische, (Aus-)Strahlung, Abrundung, Harmonie und/oder Natürlichkeit zu verleihen und/oder bestimmte (durch weitere Riech- und/oder Aromastoffe bereits vorhandene) Noten zu verstärken, kann die Gesamtmenge der Verbindung der Formel (I) auch vergleichsweise niedrig gewählt werden und z.B. bevorzugt im Bereich von 0,01 bis 5 Gew.-%, weiter bevorzugt im Bereich von 0,1 bis 2 Gew.-%, bezogen auf die Gesamtmenge der Riech- und/oder Aromastoffkomposition, liegen. Sofern innerhalb der bevorzugten Konzentrationsbereiche eine vergleichsweise niedrige Konzentration gewählt wird, kann es in Abhängigkeit von den weiteren Komponenten der jeweiligen Komposition im Einzelfall noch nicht zu der Vermittlung der hierin angegebenen Eigengeruchs- und/oder Geschmacksnoten kommen. So kann der Fachmann je nach gewünschtem Effekt der Verbindung der Formel (I) eine für die jeweilige Anwendung günstige Menge an Verbindung der Formel (I) auswählen.

Die Verbindung der Formel (I) eignet sich wegen ihrer olfaktorischen Eigenschaften vorzüglich für den Einsatz in erfindungsgemäßen Riech- und/oder Aromastoffkompositionen. Die Verbindung der Formel (I) kann vorteilhafterweise mit einer Vielzahl weiterer Riech- oder Aromastoffe kombiniert und in zahlreichen unterschiedlichen Produkten und Artikeln verwendet werden.

Vorzugsweise gilt für eine erfindungsgemäße Riech- und/oder Aromastoffkomposition, dass die Gesamtmenge an Verbindung der Formel (I) in der Riech- und/oder Aromastoffkomposition in einer sensorisch wirksamen Menge enthalten ist, vorzugsweise in einer Menge, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch und Leder-Note, vorzugsweise wenigstens eine der Geruchs- und/oder Geschmacksnoten ausgewählt aus der Gruppe bestehend aus den Noten grün, holzig, animalisch, erdig, ambrierend, fruchtig, blumig und Leder-Note, bevorzugt bestehend aus grün und fruchtig, weiter bevorzugt eine Leder-Note, zu vermitteln und/oder zu verstärken und/oder eine oder mehrere Geruchs- und/oder Geschmacksnoten eines bzw. des / der weiteren Riech- und/oder Aromastoffs/e der Riech- und/oder Aromastoffkomposition in Richtung einer oder mehrerer dieser Geruchs- und/oder Geschmacksnoten zu modifizieren.

Besonders bevorzugt ist es, wenn
(i) der bzw. einer, mehrere oder sämtliche der weiteren Riech- und/oder Aromastoffe (ebenfalls) eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch und Leder-Note, vorzugsweise eine oder mehrere der Geruchs- und/oder Geschmacksnoten ausgewählt aus der Gruppe bestehend aus den Noten grün, holzig, animalisch, erdig, ambrierend, fruchtig, blumig und Leder-Note, besonders bevorzugt ausgewählt aus der Gruppe bestehend aus den Noten fruchtig und blumig, vermitteln, modifizieren und/oder verstärken,
   oder
(ii) der bzw. einer, mehrere oder sämtliche der weiteren Riech- und/oder Aromastoffe eine oder mehrere andere Geruchs- und/oder Geschmacksnoten als die in (i) genannten vermitteln, modifizieren und/oder verstärken.

### Weiter bevorzugt ist es, wenn

(i) der bzw. einer, mehrere oder sämtliche der weiteren Riech- und/oder Aromastoffe (ebenfalls) eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, besonders bevorzugt ausgewählt aus der Gruppe bestehend aus den Noten fruchtig, blumig und Chlor-Note, vermitteln, modifizieren und/oder verstärken,
   oder
(ii) der bzw. einer, mehrere oder sämtliche der weiteren Riech- und/oder Aromastoffe eine oder mehrere andere Geruchs- und/oder Geschmacksnoten als die in (i) genannten vermitteln, modifizieren und/oder verstärken.

Beispiele für Riech- und/oder Aromastoffe, die im Rahmen der vorliegenden Erfindung vorteilhafterweise mit der Verbindung der Formel (I) kombiniert werden können, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt: Extrakte aus natürlichen Rohstoffen wie ätherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclo-pentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl) -1H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

Gemäß einer bevorzugten Ausführungsform der erfindungsgemäßen Riech- und/oder Aromastoffkomposition wird die erfindungsgemäß zu verwendende Verbindung der Formel (I) vorzugsweise mit einem oder mehreren, besonders bevorzugt mit zwei, drei, vier, fünf oder mehr, blumigen und/oder fruchtigen weiteren Riech- und/oder Aromastoffen kombiniert.

Entsprechend betrifft die vorliegende Erfindung auch eine Riechstoff- und/oder Aromastoffkomposition, die einen, zwei, drei, vier, fünf oder mehr (weitere) Riech- und/oder Aromastoffe umfasst, die eine blumige und/oder fruchtige Geruchs- und/oder Geschmacksnote vermitteln.

Dabei wird durch die erfindungsgemäß zu verwendende Verbindung der Formel (I) vorteilhafterweise (zumindest teilweise) eine geruchliche und/oder geschmackliche Verstärkung der blumigen und/oder fruchtigen Note(n) erreicht.

Blumige Riech- und/oder Aromastoffe, mit der die erfindungsgemäß zu verwendende Verbindung der Formel (I) (insbesondere in erfindungsgemäßen Riech- und/oder Aromastoffkompositionen) vorteilhaft kombiniert werden können, sind vorzugsweise ausgewählt aus der Gruppe bestehend aus:
Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol^{®}), 4-tert.-Butyl-α-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)propanol (Majantol^{®}), 3-Methyl-3-(3-methylbenzyl)-butan-2-ol, 2-Isobutyl-4-methyltetrahydro-2H-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5H-inden-5-yliden-butanal (Dupical^{®}), Vernaldehyd, 4-(4-Methyl-3-penten-1-yl)-3-cyclohexencarbaldehyd (Vertomugal^{®}), Octahydro-5-(4-methoxybutyliden)-4,7-methano-1H-inden (Mugoflor^{®}), 2,6-Dimethyl-2-heptanol (Freesiol^{®}), 1-Ethyl-1-methyl-3-phenylpropanol (Phemec^{®}), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion^{®}, Hedione^{®} high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor^{®}) und Dihydromyrcenol (2,6-Dimethyl-7-octen-2-ol).

Fruchtige Riech- und/oder Aromastoffe, mit denen die erfindungsgemäß zu verwendende Verbindung der Formel (I) vorteilhaft kombiniert werden kann, und die demnach besonders bevorzugte (weitere) Riech- und/oder Aromastoffe einer erfindungsgemäßen Riech- und/oder Aromastoffkomposition sind, sind vorzugsweise ausgewählt der Gruppe bestehend aus:
2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Vanillin, Ethylvanillin, Maltol, Ethylmaltol und deren Mischungen.

Erfindungsgemäße Riech- und/oder Aromastoffkompositionen, welche die Verbindung der Formel (I) enthalten, können in flüssiger Form, unverdünnt oder mit einem Lösungsmittel verdünnt sein bzw. werden vorteilhafterweise zur Parfümierung oder Aromatisierung eingesetzt. Bevorzugte Lösungsmittel hierfür sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin und Diacetin.

Des Weiteren können erfindungsgemäße Riech- und/oder Aromastoffkompositionen an einem Trägerstoff adsorbiert sein, der sowohl für eine feine Verteilung der Riech- und/oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff ist ebenfalls als erfindungsgemäße Riech-und/oder Aromastoffkomposition zu verstehen oder kann als erfindungsgemäßer Artikel (wie hierin weiter unten beschrieben) vorliegen.

Erfindungsgemäße Riech- und/oder Aromastoffkompositionen oder Artikel (wie hierin weiter unten beschrieben), können auch mikroverkapselt, sprühgetrocknet, als Einschluss-Komplexe oder als Extrusions-Produkte vorliegen und - im Falle einer Riech- und/oder Aromastoffkomposition - in dieser Form z. B. einem zu parfümierenden oder aromatisierenden Artikel hinzugefügt werden (wie hierin weiter unten beschrieben).

Gegebenenfalls können die Eigenschaften der derart modifizierten Kompositionen oder Artikel durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Eine Mikroverkapselung kann beispielsweise durch das sogenannte Koazervationsverfahren mit Hilfe von Kapselmaterialien z.B. aus polyurethanartigen Stoffen oder Weichgelatine, erfolgen.

Sprühgetrocknete Produkte werden vorzugsweise durch Sprühtrocknung einer die Riech- und/oder Aromastoffkomposition enthaltenden Emulsion bzw. Dispersion hergestellt, wobei als Trägerstoffe modifizierte Stärken, Proteine, Dextrin und pflanzliche Gummen verwendet werden können.

Einschluss-Komplexe können z.B. durch Eintragen von Dispersionen von der Riech- und/oder Aromastoffkomposition und Cyclodextrinen oder Harnstoffderivaten in ein geeignetes Lösungsmittel, z.B. Wasser, hergestellt werden.

Extrusions-Produkte können z.B. durch Verschmelzen der Riech- und/oder Aromastoffkompositionen mit einem geeigneten wachsartigen Stoff und durch Extrusion mit nachfolgender Erstarrung, gegebenenfalls in einem geeigneten Lösungsmittel, z.B. Isopropanol, erhalten werden.

Ein weiterer Aspekt der vorliegenden Erfindung betrifft die Verwendung einer erfindungsgemäßen Riech- und/oder Aromastoffkomposition zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens einer der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note.

Für bevorzugte Ausgestaltungen gilt hierbei das oben im Zusammenhang mit einer erfindungsgemäßen Verwendung oder erfindungsgemäßen Riech- und/oder Aromastoffkomposition Gesagte entsprechend.

Erfindungsgemäße Riech- und/oder Aromastoffkompositionen können vorteilhafterweise in konzentrierter Form, in Lösungen oder in oben beschriebener modifizierter Form verwendet werden für die Herstellung von erfindungsgemäßen parfümierten und/oder aromatisierten Artikeln (wie hierin weiter unten beschrieben) wie z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und - lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Ein weiterer Aspekt der vorliegenden Erfindung betrifft ein Verfahren zum Vermitteln, Verstärken und/oder Modifizieren einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise zum Vermitteln, Verstärken und/oder Modifizieren einer oder mehrerer Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, umfassend oder bestehend aus folgenden Schritten:
(a) Bereitstellen
   (a.1) der Verbindung der Formel (I) 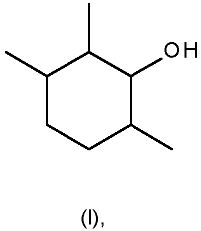 und
   (a.2) von einem, zwei, drei, vier, fünf, sechs, sieben, acht, neun, zehn oder mehr weiteren Riech- und/oder Aromastoff(en) mit einer oder mehreren Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise mit einer oder mehreren Geruchs- und/oder Geschmacksnoten ausgewählt aus der Gruppe bestehend aus den Noten grün, holzig, animalisch, erdig, ambrierend, fruchtig, blumig, Leder-Note und Chlor-Note,
(b) Hinzufügen der Verbindung der Formel (I) (a.1) zu dem/den weiteren Riech- und/oder Aromastoff(en) (a.2), in einer Menge, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note zu vermitteln, zu verstärken und/oder zu modifizieren, bevorzugt in einer Menge, die ausreicht, um eine oder mehrere Geruchs- und/oder Geschmacksnoten ausgewählt aus der Gruppe bestehend aus den Noten grün, holzig, animalisch, erdig, ambrierend, fruchtig, blumig, Leder-Note und Chlor-Note zu vermitteln, zu modifizieren und/oder zu verstärken.

Im Zusammenhang mit dem hierin beschriebenen Verfahren bzw. der Verwendung zum Vermitteln, Modifizieren und/oder Verstärken einer Geruchs- und/oder Geschmacksnote steht auch die Erkenntnis, dass die Verbindung der Formel (I) hervorragend als sogenannter Booster (Verstärker, Enhancer) fungieren kann.

Eine bevorzugte Ausführungsform betrifft demnach ein erfindungsgemäßes Verfahren zum Modifizieren und/oder Verstärken (Boosten) eines Geruchs und/oder Geschmacks, insbesondere mit einer, mehreren oder sämtlichen der Noten blumig, fruchtig und/oder holzig, umfassend oder bestehend aus folgenden Schritten:
(a) Bereitstellen
   (a.1) der Verbindung der Formel (I) 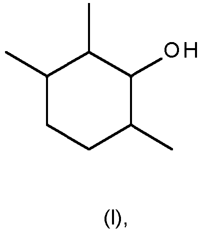 und
   (a.2) von einem oder mehreren weiteren Riech- und/oder Aromastoffen mit einer oder mehrerer der Geruchs- und/oder Geschmacksnoten ausgewählt aus der Gruppe bestehend aus blumig, fruchtig und/oder holzig,
(b) Hinzufügen der Verbindung der Formel (I) (a.1) zu dem/den weiteren Riech- und/oder Aromastoffen (a.2), in einer sensorisch wirksamen Menge, vorzugsweise in einer Menge, die ausreicht, um den Geruchs- und/oder Geschmackseindruck des bzw. der weiteren Riech- und/oder Aromastoffe (a.2), sensorisch zu modifizieren und/oder zu verstärken.

Ein weiterer Aspekt der vorliegenden Erfindung betrifft einen parfümierten und/oder aromatisierten Artikel, umfassend oder bestehend aus
(i) einer erfindungsgemäßen Riech- und/oder Aromastoffkomposition (wie hierin beschrieben),
   oder
   der Verbindung der Formel (I) 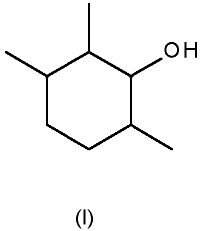 und einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
   und
(ii) einem oder mehreren weiteren Bestandteil(en), vorzugsweise wenigstens einem oder mehreren, vorzugsweise einem, zwei, drei, vier, fünf oder mehr, Zusatz-, Hilfs- und/oder Wirkstoff(en), und
wobei der Artikel ausgewählt ist aus der Gruppe bestehend aus Wasch- und Reinigungsmitteln, Hygiene- oder Pflegeprodukten, vorzugsweise im Bereich der Körper-, Haar- und Mundpflege, der Kosmetik und des Haushalts, vorzugsweise aus der Gruppe bestehend aus Oberflächenreinigern, WC-Reinigern, Allzweckreinigern, Mundwässern, Zahncremes, Zahngelen, Zahnpasten, Zahnpulvern, Zahnpflegekaugummis, Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Für bevorzugte Ausgestaltungen gilt hierbei das oben im Zusammenhang mit einer erfindungsgemäßen Verwendung, einer erfindungsgemäßen Riech- und/oder Aromastoffkomposition oder einem erfindungsgemäßen Verfahren Gesagte entsprechend.

Bevorzugt ist ein erfindungsgemäßer Artikel ausgewählt aus der Gruppe bestehend aus Wasch- und Reinigungsmitteln, Hygiene- oder Pflegeprodukten im Bereich der Körper- und Haarpflege, der Kosmetik und des Haushalts, vorzugsweise aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Außerdem gilt für einen erfindungsgemäßen parfümierten und/oder aromatisierten Artikel vorzugsweise,
dass Bestandteil (i) in einer sensorisch wirksamen Menge enthalten ist, vorzugsweise in einer Menge, die ausreicht, dass ein Verbraucher eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch und Leder-Note, vorzugsweise eine oder mehrere Geruchs- und/oder Geschmacksnoten ausgewählt aus der Gruppe bestehend aus den Noten grün, holzig, animalisch, erdig, ambrierend, fruchtig, blumig und Leder-Note, wahrnimmt,
   und/oder
dass einer, zwei, drei, vier, fünf, sechs, sieben, acht, neun, zehn oder mehr der weiteren Riech- und/oder Aromastoffe in Bestandteil (i) einen grünen, holzigen, animalischen, erdigen, ambrierenden, fruchtigen, blumigen und/oder lederigen Geruch und/oder Geschmack vermitteln, modifizieren und/oder verstärken.

Besonders bevorzugt gilt für einen erfindungsgemäßen parfümierten und/oder aromatisierten Artikel,
dass Bestandteil (i) in einer sensorisch wirksamen Menge enthalten ist, vorzugsweise in einer Menge, die ausreicht, dass ein Verbraucher eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, wahrnimmt,
   und/oder
dass einer, zwei, drei, vier, fünf, sechs, sieben, acht, neun, zehn oder mehr der weiteren Riech- und/oder Aromastoffe in Bestandteil (i) einen grünen, holzigen, animalischen, erdigen, ambrierenden, fruchtigen, blumigen, lederigen und/oder chlorartigen Geruch und/oder Geschmack vermitteln, modifizieren und/oder verstärken.

Bevorzugt ist auch, wenn der parfümierte und/oder aromatisierte Artikel eine Gesamtmenge an Verbindung der Formel (I), bezogen auf das Gesamtgewicht des Artikels, im Bereich von 0,00001 bis 10 Gew.-%, vorzugsweise 0,0001 bis 5 Gew.-%, besonders bevorzugt 0,001 bis 2 Gew.-%, weiter bevorzugt 0,005 bis 1 Gew.-%, enthält.

Die vorangehend beschriebenen Zusatz-, Hilfs- und/oder Wirkstoffe sind vorzugsweise keine Riech- und/oder Aromastoffe und sind, falls enthalten, vorzugsweise ausgewählt aus der Gruppe bestehend aus:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildner, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, (weitere) Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, fette Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Farbstoffe und farbschützende Mittel sowie Pigmente, vorzugsweise die in WO 2005/123101 genannten, Antikorrosiva, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten,
bevorzugt aus der Gruppe bestehend aus Konservierungsmitteln, anorganischen Salzen, Chelatbildnern, Tensiden, haut- und/oder haarpflegenden Mitteln, Enzymen, Emulgatoren, Fetten, fetten Ölen, Wachsen, Fettalkoholen, Silikonen, Silikonderivaten und Wasser.

Bei dem erfindungsgemäßen parfümierten und/oder aromatisierten Artikel, wie vorangehend beschrieben, handelt es sich gemäß einer bevorzugten Ausgestaltung um eine der Mundpflege dienende (erfindungsgemäße) Zubereitung.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiss, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Erfindungsgemäße Zubereitungen können z.B. als Halbfertigware oder als Würzmischung vorliegen.

Hierin beschriebene Zubereitungen können insbesondere als Halbfertigware zur Herstellung weiterer der Ernährung oder dem Genuss dienenden Zubereitungen dienen, insbesondere in sprühgetrockneter Form. Hierin beschriebene Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Mundpflege dienende erfindungsgemäße Zubereitungen können in Mengen von 5 bis 99,9999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,9 Gew.-%, vorzugsweise 5 bis 80 Gew.-%, bezogen auf das Gesamtgewicht der Zubereitung, aufweisen.

Erfindungsgemäße Zubereitungen (als Beispiele erfindungsgemäßer Artikel) werden gemäß einer bevorzugten Ausgestaltung hergestellt, indem Verbindungen als Substanz, als Lösung (z. B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Mundpflege dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (vorzugsweise Halbfertigware) überführt werden.

Die erfindungsgemäßen sprühgetrockneten festen Zubereitungen (als weiteres Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 Gew.-% Trägerstoffe, insbesondere Maltodextrin und/oder Stärke, 5 bis 40 Gew.-% Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen die Verbindung der Formel (I) und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können, je nach Matrix, die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung werden Verbindungen zunächst mit einem oder mehreren Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass die Verbindung der Formel (I) verzögert von der Matrix freigegeben wird, so dass eine langanhaltende Wirkung erzielt wird. Besonders bevorzugt ist insoweit eine Fett-, Wachs-, Polysaccharid- oder Proteinmatrix.

Als weitere Bestandteile für hierin beschriebene, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder - pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Zahnpflegemittel (als Basis für der Mundpflege dienende erfindungsgemäße Zubereitungen) umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende erfindungsgemäße Zubereitungen) umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigenzien.

Ein weiterer Aspekt der vorliegenden Erfindung betrifft ein Verfahren zum Parfümieren und/oder Aromatisieren eines Artikels, umfassend oder bestehend aus folgenden Schritten:
(a) Bereitstellen
   (a.1) einer Riech- und/oder Aromastoffkomposition (wie hierin beschrieben),
      oder
   (a.2) der Verbindung der Formel (I) 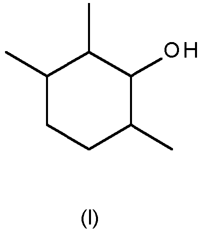 und optional einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
      und
(b) Hinzufügen der Riech- und/oder Aromastoffkomposition (a.1) bzw. der Verbindung / Riechstoffe (a.2) zu dem zu parfümierenden und/oder aromatisierenden Artikel, in einer Menge, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, zu vermitteln, zu verstärken und/oder zu modifizieren.

Weiterhin betrifft die vorliegende Erfindung ein Verfahren zum Parfümieren von Haaren, Haut, textilen Fasern, Oberflächen und/oder Raumluft umfassend oder bestehend aus folgenden Schritten:
(a) Bereitstellen
   (a.1) einer Riech- und/oder Aromastoffkomposition (wie hierin beschrieben), vorzugsweise enthaltend ein Tensid oder eine Tensidmischung,
      oder
   (a.2) der Verbindung der Formel (I) 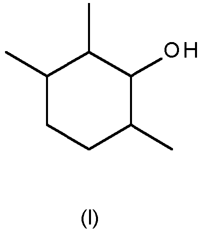
      und optional einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
      und zudem bevorzugt eines Tensids oder einer Tensidmischung,
         oder
   (a.3) eines erfindungsgemäßen Artikels (wie hierin beschrieben), vorzugsweise enthaltend ein Tensid oder eine Tensidmischung,
      und
(b) Auftragen bzw. Einbringen der Riech- und/oder Aromastoffkomposition (a.1) bzw. der Verbindung / Riechstoffe (a.2) bzw. des Artikels (a.3) auf die zu parfümierende(n) Haare bzw. Haut bzw. Fasern bzw. Oberfläche bzw. in die zu parfümierende Raumluft in einer Menge, die ausreicht, dass der Verbraucher eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens eine der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, wahrnimmt.

Für bevorzugte Ausgestaltungen der hierin beschriebenen Verfahren gilt wiederum das im Zusammenhang mit erfindungsgemäßen Verwendungen oder erfindungsgemäßen Riech- und/oder Aromastoffkompositionen oder erfindungsgemäßen Artikeln Gesagte entsprechend.

Des Weiteren gilt das im Rahmen einer hierin beschriebenen Ausführungsform der vorliegenden Erfindung Gesagte selbstverständlich jeweils auch für andere hierin beschriebene Ausführungsformen. Dementsprechend sind die hierin beschriebenen Ausführungsformen beliebig - soweit für den Fachmann sinnvoll - miteinander kombinierbar.

Nachfolgend wird die Erfindung anhand von Beispielen näher erläutert. Sofern nicht anders angegeben, beziehen sich alle Angaben auf das Gewicht.
Verwendete Abkürzungen: DPG = Dipropylenglycol, TEC = Triethylcitrat

### Beispiel 1: Herstellung von 2,3,6-Trimethylcyclohexanol (Verbindung der Formel (I)

In einen 250 ml Autoklaven werden 138 g 2,5,6-Trimethylcyclohex-2-en-1-on in 50 g Isopropanol mit 2 g Raney-Nickel bei 10 bar und 120°C 6 h hydiert. Nach Abkühlung wirde der Katalysator abfiltriert und anschließend wird eingeengt. Das Rohprodukt (141 g) wird an einer 30 cm Vigreux-Kolonne im Vakuum fraktioniert destilliert.
Ausbeute: 138 g (97,2 % d. Th.)
Sdp.: 95°-98°C / 2 mbar
MS: m/z (%) = 142(1-12), 124(20-57), 109(50-85), 95(56-98), 85(96-100), 71(62-100), 55(53-80), 41(48-77)

### Beispiel 2: Parfümkomposition (Riechstoffkomposition)

| IRIS SAFFIAN | |
|---|---|
| Total in g (18 Teile): | 1.000,00 |
| ALDEHYD C14 SOG | 5 |
| ALLYLIONON | 4 |
| AMBROCENIDE^{®} 10 DPG 10% DPG | 10 |
| AMYRISOEL | 100 |
| CEDERNHOLZOEL | 200 |
| ELEMI RESIN 70% IN BB | 275 |
| GUAJYLACETAT | 5 |
| IONON ALPHA | 80 |
| IRALDEIN GAMMA | 80 |
| IRISNITRIL 1% DPG | 25 |
| KAROTTENSAMENOEL | 5 |
| MADRANOL^{®} | 100 |
| MYRRHEN ABSOLUE | 10 |
| NONADIENOL-2,6 1% DPG | 10 |
| PATCHOULIOEL ENTF. | 5 |
| PERUBALSAMOEL ED | 3 |
| TABANON 10% DPG | 3 |
| VERTOFIX | 80 |

Nach Meinung der Parfümeure wird diese Parfümkomposition durch den Zusatz von 3 Gew.-% an Verbindung der Formel (I) (z.B. Produkt aus Beispiel 1, 1%ige Lösung in DPG) abgerundeter und harmonischer, wobei eine Leder-Note hinzukommt und die holzigen und blumigen Aspekte verstärkt werden. Die erfindungsgemäße Kombination bzw. Verwendung verleiht der Komposition einen eigenen Charakter und verbindet die unterschiedlichen geruchlichen Elemente.

### Beispiel 3: Parfümkomposition (Riechstoffkomposition)

| CRISTAL CHEAP | |
|---|---|
| Total in g (39 Teile): | 1.000,00 |
| ALDEHYD C14 SOG. | 3 |
| BASILIKUMOEL COMORES TYP E | 0,5 |
| BENZYLSALICYLAT | 45 |
| CEDRYLACETAT | 5 |
| CITRAL FF | 2 |
| CITRONE RCO | 60 |
| CITRONELLOL 950 | 12 |
| CITRORANGE BASE COLIPA | 2 |
| DIHYDROJASMON | 4 |
| DIHYDROMYRCENOL | 10 |
| DIPROPYLENGLYCOL | 201,5 |
| EVERNYL | 2 |
| FARENAL^{®} | 0,5 |
| GALBANUMOEL | 1 |
| GALBASCONE 10% DPG | 9 |
| GERANIOL SUPRA | 5 |
| GERANYLACETAT PUR | 3 |
| HEDION | 280 |
| HELIONAL | 25 |
| HERBAFLORAT | 5 |
| HEXYLZIMTALDEHYD ALPHA | 45 |
| INDOLENE 50 RIZINUSOEL 10% DPG | 10 |
| ISORALDEIN 70 | 40 |
| JASMIN 61 TYPE BASE | 10 |
| JASMOPYRAN | 4 |
| LAVENDELOEL MT. BLANC 40/42% | 2 |
| LILIAL | 35 |
| LINALOOL | 30 |
| LINALYLACETAT | 25 |
| MACROLIDE^{®} SUPRA | 3 |
| MYSORANE^{®} BASE | 20 |
| NEOFOLIONE 10% DPG | 2,5 |
| ORANGENOEL FLORIDA TYP VALENZIA | 25 |
| PATCHOULI LIGHT | 2 |
| PHENYLETHYLALKOHOL | 20 |
| SANDRANOL^{®} | 5 |
| VETIVERYLIA BASE | 5 |
| YLANG MC TYPE BASE | 40 |
| ZIBETH ABO394E 10% DPG | 1 |

Nach Meinung der Parfümeure erwacht diese Parfümkomposition durch den Zusatz von 1 Gew.-% an Verbindung der Formel (I) (z.B. Produkt aus Beispiel 1, 1%ige Lösung in DPG) zu neuem Leben. Der Eindruck von Fruchtigkeit und Blumigkeit wird erheblich verstärkt. Die Komposition wirkt harmonischer, wobei eine natürliche Note hinzukommt. Das Produkt aus Beispiel 1 (d.h. das Isomerengemisch der Verbindung der Formel (I)) verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 4: Parfümkomposition (Riechstoffkomposition)

| MAGIQUE | |
|---|---|
| Total in g (30 Teile): | 1.000,00 |
| ALDEHYD C14 SOG | 5 |
| AMBERWOOD^{®} F | 15 |
| AMBROCENIDE^{®} 10 DPG 10% DPG | 15 |
| BENZYLACETAT | 10 |
| BENZYLSALICYLAT | 95 |
| BERGAMOT IDENTOIL^{®} COLOURLESS | 25 |
| CASHMERAN | 5 |
| CEDERNHOLZOEL CHIN. | 20 |
| DAMASCONE DELTA 10% DPG | 10 |
| DIPROPYLENGLYCOL | 195 |
| ELEMIOEL | 5 |
| ETHYLVANILLIN | 5 |
| FRAMBINON^{®} | 15 |
| GERANIOL 60 | 10 |
| GLOBALIDE^{®} | 120 |
| HEDION | 65 |
| HELIONAL | 10 |
| IONON BETA | 20 |
| ISO E SUPER | 95 |
| JASMIN ABS. BASE | 25 |
| LILIAL | 95 |
| METHYLBENZOAT 10% DPG | 5 |
| ORANGENOEL | 15 |
| PATCHOULIOEL ECHT | 25 |
| PHENYLETHYLALKOHOL | 35 |
| ROSENOXID HIGH CIS 10% DPG | 5 |
| VANILLIN | 10 |
| YSAMBER^{®} K | 35 |
| ZIBETH ABO394E 10% DPG | 5 |
| ZIMTALKOHOL | 5 |

Nach Meinung der Parfümeure gewinnt die Parfümkomposition durch den Zusatz von 1 Gew.-% an Verbindung der Formel (I) (z.B. Produkt aus Beispiel 1, 1%ige Lösung in DPG) an Ausstrahlung. Der Eindruck einer feinen Leder-Note wird erheblich verstärkt. Die Komposition wirkt weicher, wobei eine natürliche Note hinzukommt. Das Produkt aus Beispiel 1 (d.h. das Isomerengemisch der Verbindung der Formel (I)) verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 5: Shampoo

Das erfindungsgemäß zu verwendende Produkt aus Beispiel 1 (d.h. das Isomerengemisch der Verbindung der Formel (I)) wurde in einer Dosierung von 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| Cocamidopropylbetain (z.B. Dehyton K, Fa. Cognis Deutschland GmbH) | 2% |
| Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden zwei Haarsträhnchen gemeinsam für 2 Minuten gewaschen und anschließend 20 Sekunden unter fließendem handwarmem Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

Geruchsbeschreibung beider Haarsträhnen: Stark holzige, animalische, erdig mit ambrierender Note.

### Beispiel 6: Weichspüler

Die Parfümkomposition aus Beispiel 2 (nach Zusatz von 1 Gew.-% einer 1%igen Lösung des Produktes aus Beispiel 1 in DPG) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% (z.B. Rewoquat WE 18, Fa. Witco Surfactants GmbH) | 5,5% |
| Alkyldimethylbenzylammoniumchlorid, ca. 50% (z.B. Preventol R50, Fa. Bayer AG) | 0,2% |
| Farblösung, ca. 1%-ig | 0,3% |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% der Parfümkomposition aus Beispiel 2 umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung beider Stofflappen: Abgerundet und harmonisch im Geruch, mit einer feinen Leder Note.

### Beispiel 7: Waschpulver

Die Parfümölkomposition aus Beispiel 3 (nach Zusatz von 1 Gew.-% einer 1%igen Lösung des Produktes aus Beispiel 1 in DPG) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer DOW CORNING 2-4248S POWDERED ANTIFOAM, Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 ([[(Phosphonomethyl)imino]bis[(ethylennitrilo)bis (methylen)]]tetrakis-phosphonsäure, Natriumsalz) | 2,8 % |
| Optischer Aufheller | 0,2 % |
| Na-Sulfat | 6,5 % |
| Protease | 0,4 % |
| Natriumperborat-tetrahydrat | 22,0 % |
| TAED | 1,0 % |

Zwei Stofflappen wurden mit 370 g einer 1%-igen wässrigen Waschpulverlauge auf Basis des 0,4 Gew.-% der Parfümölkomposition aus Beispiel 3 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung beider Stofflappen: Fruchtig und blumig harmonisch, mit einer natürlichen holzigen Note.

### Beispiel 8: Zahnpasta

Zahnputz-Zusammensetzung umfassend eine Riech- und Aromastoff-Komposition mit Wintergrün-Charakter

### Beispiel 8.1 (Vergleichsbeispiel):

Durch Vermischen von

| | | |
|---|---|---|
| 17 | Gew.-% | Pfefferminzöl Mentha arvensis, rektifiziert |
| 17,5 | Gew.-% | Pfefferminzöl Mentha piperita Typ Willamette |
| 25 | Gew.-% | Methylsalicylat |
| 40 | Gew.-% | I-Menthol |
| 0,5 | Gew.-% | Menthancarbonsäure-N-(4-methoxyphenyl)-amid |

wurde eine Riech- und Aromastoff-Komposition für Zahnputz-Zusammensetzungen mit Wintergrün-Charakter hergestellt.

### Beispiel 8.2:

Durch Vermischen von

| | | |
|---|---|---|
| 17 | Gew.-% | Pfefferminzöl Mentha arvensis, rektifiziert |
| 17,5 | Gew.-% | Pfefferminzöl Mentha piperita Typ Willamette |
| 25 | Gew.-% | Methylsalicylat |
| 40 | Gew.-% | I-Menthol |
| 0,3 | Gew.-% | Menthancarbonsäure-N-(4-methoxyphenyl)-amid |
| 0,2 | Gew.-% | Produkt aus Beispiel 1 (Verbindung der Formel (I)) |

wurde eine erfindungsgemäße Riech- und Aromastoff-Komposition mit einem Anteil von 0,2 Gew.-% der Verbindung der Formel (I) für Zahnputz-Zusammensetzungen mit Wintergrün-Charakter hergestellt.

Die Riech- und Aromastoff-Kompositionen gemäß Beispiel 8.1 (für eine Vergleichs-Zahnputz-Zusammensetzung) und Beispiel 8.2 (für eine erfindungsgemäße Zahnputz-Zusammensetzung) wurden jeweils mit einer Konzentration von 1,2 Gew.-% in eine jeweilige Standard-Zahnpasta-Masse auf Kieselsäure-Basis eingearbeitet, bezogen auf die Gesamtmasse der resultierenden Zahnpasta.

Die daraus resultierenden Zahnpasten wurden von einem sensorisch geschulten Expertenpanel unter Praxisbedingungen getestet. Die sensorische Bewertung für die Zahnpasta enthaltend eine Riech- und Aromastoff-Komposition gemäß Beispiel 8.2 ergab eine ausgeprägte frisch-pfefferminzige Wintergrün-Note mit einem sehr starken und ausgeprägten, sehr lang anhaltenden, kühlenden Frischeempfinden und einer leichten Chlor-Note, die eine antibakterielle Wirksamkeit der Zahnpasta vermittelt. Die Chlor-Note war in der Vergleichs-Zahnputz-Zusammensetzung 8.1 nicht wahrnehmbar.

### Beispiel 9: Mundwasser

Mundwasser bzw. Mundwasserkonzentrat umfassend eine Riech- und Aromastoff-Komposition mit Eucalyptus-Charakter

### Beispiel 9.1 (Vergleichsbeispiel):

Durch Vermischen von

| | | |
|---|---|---|
| 10 | Gew.-% | Anethol |
| 17,5 | Gew.-% | Eucalyptusöl (70-75% 1,8-Cineol) |
| 17,5 | Gew.-% | 1,8-Cineol (Eucalyptol) |
| 54,4 | Gew.-% | *I*-Menthol |
| 0,6 | Gew.-% | Menthancarbonsäure-N-(4-methoxyphenyl)-amid |

wurde eine Riech- und Aromastoff-Komposition für den Einsatz in Mundwässern bzw. Mundwasserkonzentraten hergestellt.

### Beispiel 9.2:

Durch Vermischen von

| | | |
|---|---|---|
| 10 | Gew.-% | Anethol |
| 17,5 | Gew.-% | Eucalyptusöl (70-75% 1,8-Cineol) |
| 17,5 | Gew.-% | 1,8-Cineol (Eucalyptol) |
| 54,4 | Gew.-% | *I*-Menthol |
| 0,3 | Gew.-% | Menthancarbonsäure-N-(4-methoxyphenyl)-amid |
| 0,3 | Gew.-% | Produkt aus Beispiel 1 (Verbindung der Formel (I)) |

wurde eine Riech- und Aromastoff-Komposition mit einem Anteil von 0,2 Gew.-% der Verbindung der Formel (I) für den Einsatz in Mundwässern bzw. Mundwasserkonzentraten hergestellt.

Die Riech- und Aromastoff-Kompositionen gemäß Beispiel 9.1 (für ein Vergleichs-Mundwasser bzw. ein Vergleichs-Mundwasserkonzentrat) und Beispiel 9.2 (für ein erfindungsgemäßes Mundwasser bzw. ein erfindungsgemäßes Mundwasserkonzentrat) wurden jeweils mit einer Konzentration von 0,45 Gew.-% in jeweils ein gebrauchsfertiges Mundwasser bzw. jeweils mit einer Konzentration von 3 Gew.-% in jeweils ein gebrauchsfertiges Mundwasserkonzentrat eingearbeitet, bezogen auf die Gesamtmasse des resultierenden Mundwassers bzw. des resultierenden Mundwasserkonzentrates.

Die daraus resultierenden Mundwasser bzw. Mundwasserkonzentrate wurden von einem sensorisch geschulten Expertenpanel unter Praxisbedingungen getestet. Die sensorische Bewertung für das Mundwasser/Mundwasserkonzentrat, enthaltend eine Riech- und Aromastoff-Komposition gemäß Beispiel 9.2, ergab eine starke, angenehm frische Eucalyptus-Note mit einem sehr lang anhaltenden Frischeempfinden, das auch nach Verwendung des Mundwassers/Mundwasserkonzentrates noch über eine Zeit von weit über 30 Minuten anhielt. Zudem kann eine Chlor-Note deutlich wahrgenommen werden, welche einen Eindruck von Hygiene und antibakterieller Wirksamkeit vermittelt. Das Mundwasser/Mundwasserkonzentrat, enthaltend eine Riech- und Aromastoff-Komposition gemäß Beispiel 9.1 weist im Gegensatz dazu keine wahrnehmbare Chlor-Note auf.

### Beispiel 10: Allzweckreiniger

| **Material** | **Hersteller** | **Chemischer Name** | **Funktion** | **Gew. -%** |
|---|---|---|---|---|
| Entionisiertes Wasser | | Water | Lösungsmittel | 59,6 |
| Mergal K9N | Troy Chemie, Seelze | 5-Chloro-2-methyl-3-(2H)-isothiazolone und 2-methyl-3-(2H)-isothiazolone | Konservierungs mittel | 0,1 |
| Trinatriumcitrat Dihydrat | Verschiedene | Tri Sodium Citrate Dihydrate | Komplexbildner | 3,0 |
| Zetesol NL-2 | Zschimmer & Schwarz, Deutschland | Fatty alcohol C12-14-sulfate, Sodium | Anionisches Tensid | 30,0 |
| Imbentin C/125/055 | Dr. W. Kolb AG Chem. | Fatty alcohol C12-C15, 8EO | Nichtionisches Tensid | 5,0 |
| Ethanol 96% | Verschiedene | Ethanol | Lösungsmittel | 2,0 |
| Parfümkomposition aus Beispiel 3 | Symrise | | Parfum (Fragrance) | 0,3 |

Bei einer Dosierung von 0.3 Gew.-% der Parfümkomposition aus Beispiel 3 im Allzweckreiniger ergibt sich folgender Befund:
Ein Zusatz von 1 Gew.-% einer 1%igen Lösung des Produktes aus Beispiel 1 in DPG (bezogen auf das Gesamtgewicht der Parfümkomposition aus Beispiel 3) zum Allzweckreiniger bewirkt eine erhebliche Verstärkung des Eindrucks von Fruchtigkeit und Blumigkeit. Zudem kann nach dem Zusatz der Verbindung der Formel (I) eine dezente Chlor-Skatol-Note wahrgenommen werden, welche den Eindruck von Sauberkeit und Hygiene vermittelt.

## Patentansprüche

1. Verwendung der Verbindung der Formel (I), 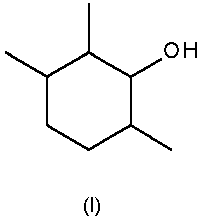 als Riech- und/oder Aromastoff zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise einer oder mehrerer der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note.

2. Riech- und/oder Aromastoffkomposition umfassend oder bestehend aus der Verbindung der Formel (I) 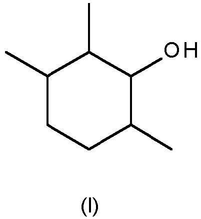
und einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt, und
wobei die Gesamtmenge an Verbindung der Formel (I) in der Riech- und/oder Aromastoffkomposition in einer Menge enthalten ist, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens eine der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, zu vermitteln und/oder zu verstärken und/oder eine oder mehrere Geruchs- und/oder Geschmacksnoten eines bzw. des/der weiteren Riech- und/oder Aromastoffs/e der Riech- und/oder Aromastoffkomposition in Richtung einer oder mehrerer dieser Geruchs- und/oder Geschmacksnoten zu modifizieren.

3. Riech- und/oder Aromastoffkomposition nach Anspruch 2, wobei die Gesamtmenge an Verbindung der Formel (I), bezogen auf das Gesamtgewicht der Riech- und/oder Aromastoffkomposition, im Bereich von 0,0001 bis 90 Gew.-% liegt.

4. Riech- und/oder Aromastoffkomposition nach einem der Ansprüche 2 oder 3, wobei
(i) der bzw. einer, mehrere oder sämtliche der weiteren Riech- und/oder Aromastoffe (ebenfalls) eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, besonders bevorzugt ausgewählt aus der Gruppe bestehend aus den Noten fruchtig, blumig und Chlor-Note, vermitteln, modifizieren und/oder verstärken,
oder wobei
(ii) der bzw. einer, mehrere oder sämtliche der weiteren Riech- und/oder Aromastoffe eine oder mehrere andere Geruchs- und/oder Geschmacksnoten als die in (i) genannten vermitteln, modifizieren und/oder verstärken.

5. Verwendung einer Riech- und/oder Aromastoffkomposition nach einem der Ansprüche 2 bis 4 zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens einer der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note.

6. Verfahren zum Vermitteln, Verstärken und/oder Modifizieren einer oder mehrerer Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise zum Vermitteln, Verstärken und/oder Modifizieren einer oder mehrerer Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, umfassend oder bestehend aus folgenden Schritten:
(a) Bereitstellen
(a.1) der Verbindung der Formel (I) 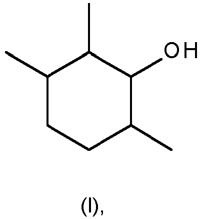 und
(a.2) von einem, zwei, drei, vier, fünf, sechs, sieben, acht, neun, zehn oder mehr weiteren Riech- und/oder Aromastoff(en) mit einer oder mehreren Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise mit einer oder mehreren Geruchs- und/oder Geschmacksnoten ausgewählt aus der Gruppe bestehend aus den Noten grün, holzig, animalisch, erdig, ambrierend, fruchtig, blumig, Leder-Note und Chlor-Note,
(b) Hinzufügen der Verbindung der Formel (I) (a.1) zu dem/den weiteren Riech- und/oder Aromastoff(en) (a.2), in einer Menge, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note zu vermitteln, zu verstärken und/oder zu modifizieren, bevorzugt in einer Menge, die ausreicht, um eine oder mehrere Geruchs- und/oder Geschmacksnoten ausgewählt aus der Gruppe bestehend aus den Noten grün, holzig, animalisch, erdig, ambrierend, fruchtig, blumig, Leder-Note und Chlor-Note zu vermitteln, zu modifizieren und/oder zu verstärken.

7. Parfümierter und/oder aromatisierter Artikel, umfassend oder bestehend aus
(i) einer Riech- und/oder Aromastoffkomposition nach einem der Ansprüche 2 bis 4,
oder
der Verbindung der Formel (I) 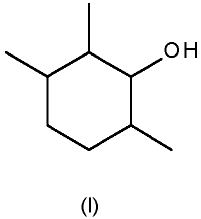 und einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
und
(ii) einem oder mehreren weiteren Bestandteil(en), vorzugsweise wenigstens einem oder mehreren, vorzugsweise einem, zwei, drei, vier, fünf oder mehr, Zusatz-, Hilfs- und/oder Wirkstoff(en), und
wobei der Artikel ausgewählt ist aus der Gruppe bestehend aus Wasch- und Reinigungsmitteln, Hygiene- oder Pflegeprodukten, vorzugsweise im Bereich der Körper-, Haar- und Mundpflege, der Kosmetik und des Haushalts, vorzugsweise aus der Gruppe bestehend aus Oberflächenreinigern, WC-Reinigern, Allzweckreinigern, Mundwässern, Zahncremes, Zahngelen, Zahnpasten, Zahnpulvern, Zahnpflegekaugummis, Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und - lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

8. Parfümierter und/oder aromatisierter Artikel nach Anspruch 7, wobei Bestandteil (i) in einer Menge enthalten ist, die ausreicht, dass ein Verbraucher eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, wahrnimmt.

9. Parfümierter und/oder aromatisierter Artikel nach einem der Ansprüche 7 oder 8, wobei die Gesamtmenge an Verbindung der Formel (I), bezogen auf das Gesamtgewicht des Artikels, im Bereich von 0,00001 bis 10 Gew.-%, vorzugsweise 0,0001 bis 5 Gew.-%, besonders bevorzugt 0,001 bis 2 Gew.-%, weiter bevorzugt 0,005 bis 1 Gew.-%, liegt.

10. Verfahren zum Parfümieren und/oder Aromatisieren eines Artikels, umfassend oder bestehend aus folgenden Schritten:
(a) Bereitstellen
(a.1) einer Riech- und/oder Aromastoffkomposition nach einem der Ansprüche 2 bis 4,
oder
(a.2) der Verbindung der Formel (I) 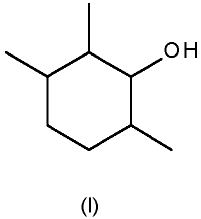 und optional einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
und
(b) Hinzufügen der Riech- und/oder Aromastoffkomposition (a.1) bzw. der Verbindung / Riechstoffe (a.2) zu dem zu parfümierenden und/oder aromatisierenden Artikel, in einer Menge, die ausreicht, um eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise eine oder mehrere der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, zu vermitteln, zu verstärken und/oder zu modifizieren.

11. Verfahren zum Parfümieren von Haaren, Haut, textilen Fasern, Oberflächen und/oder Raumluft umfassend oder bestehend aus folgenden Schritten:
(a) Bereitstellen
(a.1) einer Riech- und/oder Aromastoffkomposition nach einem der Ansprüche 2 bis 4, vorzugsweise enthaltend ein Tensid oder eine Tensidmischung,
oder
(a.2) der Verbindung der Formel (I) 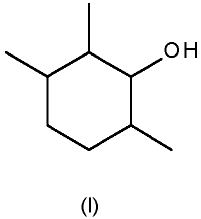
und optional einem, zwei, drei, vier, fünf, sechs, sieben, acht, neun, zehn oder mehr weiteren Riech- und/oder Aromastoff(en), wobei das Gewichtsverhältnis der Gesamtmenge an Verbindung der Formel (I) zu der Gesamtmenge an weiterem/n Riech- und/oder Aromastoff(en) im Bereich von 1 : 1000 bis 1 : 0,1, vorzugsweise von 1 : 1000 bis 1 : 0,5, liegt,
und zudem bevorzugt eines Tensids oder einer Tensidmischung,
oder
(a.3) eines Artikels nach einem der Ansprüche 7 bis 9, vorzugsweise enthaltend ein Tensid oder eine Tensidmischung,
und
(b) Auftragen bzw. Einbringen der Riech- und/oder Aromastoffkomposition (a.1) bzw. der Verbindung / Riechstoffe (a.2) bzw. des Artikels (a.3) auf die zu parfümierende(n) Haare bzw. Haut bzw. Fasern bzw. Oberfläche bzw. in die zu parfümierende Raumluft in einer Menge, die ausreicht, dass der Verbraucher eine oder mehrere Geruchs- und/oder Geschmacksnoten ausgewählt aus der Gruppe bestehend aus den Noten grün, krautig, frisch, fruchtig, blumig, holzig, animalisch, süß, erdig, ambrierend, fettig, metallisch, balsamisch, Leder-Note und Chlor-Note, vorzugsweise wenigstens eine der Geruchs- und/oder Geschmacksnoten ausgewählt aus der Gruppe bestehend aus den Noten grün, holzig, animalisch, erdig, ambrierend, fruchtig, blumig, Leder-Note und Chlor-Note, wahrnimmt.

## Claims

1. Use of a compound of formula (I), 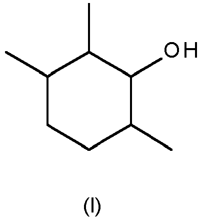 as fragrance and/or flavoring for imparting, modifying and/or enhancing one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably one or more of the scent and/or flavor notes selected from the group consisting of the notes green, woody, animalistic, earthy, ambery, fruity, floral, leather note and chlorine note.

2. Fragrance and/or flavoring composition comprising or consisting of a compound of formula (I) 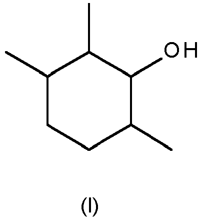
and one, two, three, four, five, six, seven, eight, nine, ten or more further fragrance(s) and/or flavoring(s), wherein the weight ratio of the total amount of compound of formula (I) to the total amount of further fragrance(s) and/or flavoring(s) is in the range of 1 : 1000 to 1 : 0.1, preferably 1 : 1000 to 1 : 0.5, and
wherein the total amount of the compound of formula (I) in the fragrance and/or flavoring composition is contained in an amount sufficient to impart and/or enhance one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably at least one of the scent and/or flavor notes selected from the group consisting of the notes green, woody, animalistic, earthy, ambery, fruity, floral, leather note and chlorine note, and/or to modify one or more scent and/or flavor notes of a or the further fragrance(s) and/or flavoring(s) of the fragrance and/or flavoring composition in the direction of one or more of said scent and/or flavor notes.

3. Fragrance and/or flavoring composition according to claim 2, wherein the total amount of the compound of formula (I), based on the total weight of the fragrance and/or flavoring composition, is in the range of 0.0001 to 90% by weight.

4. Fragrance and/or flavoring composition according to one of the claims 2 or 3, wherein
(i) the or one, several or all of the further fragrances and/or flavorings (also) impart, modify and/or enhance one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably one or more of the scent and/or flavor notes selected from the group consisting of the notes green, woody, animalistic, earthy, ambery, fruity, floral, leather note and chlorine note, particularly preferably selected from the group consisting of the notes fruity, floral and chlorine note,
or wherein
(ii) the or one, several or all of the further fragrances and/or flavorings impart, modify and/or enhance one or more scent and/or flavor notes other than those mentioned in (i).

5. Use of a fragrance and/or flavoring composition according to one of claims 2 to 4 for imparting, modifying and/or enhancing one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably at least one of the scent and/or flavor notes selected from the group consisting of the notes green, woody, animalistic, earthy, ambery, fruity, floral, leather note and chlorine note.

6. Method for imparting, enhancing and/or modifying one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably for imparting, enhancing and/or modifying one or more scent and/or flavor notes selected from the group consisting of the notes green, woody, animalistic, earthy, ambery, fruity, floral, leather note and chlorine note, comprising or consisting of the following steps:
(a) Providing
(a.1) the compound of formula (I) 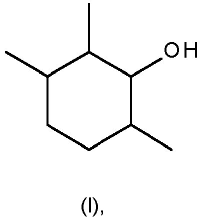 and
(a.2) one, two, three, four, five, six, seven, eight, nine, ten or more further fragrance(s) and/or flavoring(s) with one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably with one or more scent and/or flavor notes selected from the group consisting of the notes green, woody, animalistic, earthy, ambery, fruity, floral, leather note and chlorine note,
(b) adding the compound of formula (I) (a.1) to the further fragrance(s) and/or flavoring(s) (a.2), in an amount sufficient to impart, enhance and/or modify one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably in an amount sufficient to impart, modify and/or enhance one or more scent and/or flavor notes selected from the group consisting of the notes green, woody, animalistic, earthy, ambery, fruity, floral, leather note and chlorine note.

7. Perfumed and/or flavored article, comprising or consisting of
(i) a fragrance and/or flavoring composition according to any of the claims 2 to 4, or
the compound of formula (I) 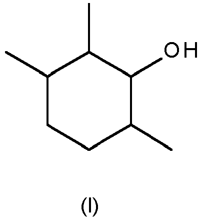 and one, two, three, four, five, six, seven, eight, nine, ten or more further fragrance(s) and/or flavoring(s), wherein the weight ratio of the total amount of the compound of formula (I) to the total amount of further fragrance(s) and/or flavoring(s) is in the range of 1 : 1000 to 1 : 0.1, preferably 1 : 1000 to 1 : 0.5,
and
(ii) one or more further component(s), preferably at least one or several, preferably one, two, three, four, five or more, additive(s), excipient(s) and/or active substance(s), and
wherein the article is selected from the group consisting of laundry and cleaning detergents, hygiene or care products, preferably in the field of body, hair and oral care, cosmetics and household, preferably from the group consisting of surface cleaners, WC cleaners, all-purpose cleaners, mouthwashes, toothpastes, tooth gels, dentifrices, tooth powders, tooth care chewing gums, perfume extracts, eau de parfums, eau de toilettes, shaving lotions, eau de colognes, pre-shave products, splash colognes, perfumed refreshing wipes, acidic, alkaline or neutral detergents, textile fresheners, ironing aids, liquid laundry detergents, powdery laundry detergents, laundry pretreatment agents, fabric softeners, washing soaps, washing tablets, disinfectants, surface disinfectants, air fresheners, aerosol sprays, waxes and polishes, body care products, hand creams and lotions, foot creams and lotions, depilatory creams and lotions, after-shave creams and lotions, tanning creams and lotions, hair care products, deodorants, antiperspirants, decorative cosmetic products, candles, lamp oils, incense sticks, insecticides, repellents and fuels.

8. Perfumed and/or flavored article according to claim 7, wherein component (i) is contained in an amount sufficient for a consumer to perceive one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably one or more scent and/or flavor notes selected from the group consisting of the notes green, woody, animalistic, earthy, ambery, fruity, floral, leather note and chlorine note.

9. Perfumed and/or flavored article according to any of the claims 7 or 8, wherein the total amount of the compound of formula (I), based on the total weight of the article, is in the range of 0.00001 to 10% by weight, preferably 0.0001 to 5% by weight, particularly preferably 0.001 to 2% by weight, more preferably 0.005 to 1% by weight.

10. Method for perfuming and/or flavoring an article, comprising or consisting of the steps of:
(a) Providing
(a.1) a fragrance and/or flavoring composition according to any of the claims 2 to 4,
or
(a.2) the compound of formula (I) 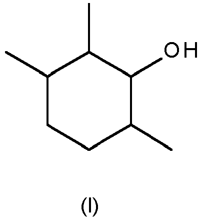 and optionally one, two, three, four, five, six, seven, eight, nine, ten or more further fragrance(s) and/or flavoring(s), wherein the weight ratio of the total amount of the compound of formula (I) to the total amount of further fragrance(s) and/or flavoring(s) is in the range from 1 : 1000 to 1 : 0.1, preferably from 1 : 1000 to 1 : 0.5,
and
(b) adding the fragrance and/or flavoring composition (a.1) or the compound / fragrances (a.2) to the article to be perfumed and/or flavored, in an amount sufficient to impart, enhance and/or modify one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably one or more of the scent and/or flavor notes selected from the group consisting of the notes green, woody, animalistic, earthy, ambery, fruity, floral, leather note and chlorine note.

11. Method for perfuming hair, skin, textile fibers, surfaces and/or room air comprising or consisting of the following steps:
(a) Providing
(a.1) a fragrance and/or flavoring composition according to any of the claims 2 to 4, preferably containing a surfactant or a surfactant mixture,
or
(a.2) the compound of formula (I) 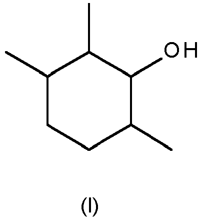
and optionally one, two, three, four, five, six, seven, eight, nine, ten or more further fragrance(s) and/or flavoring(s), wherein the weight ratio of the total amount of the compound of formula (I) to the total amount of further fragrance(s) and/or flavoring(s) is in the range from 1 : 1000 to 1 : 0.1, preferably from 1 : 1000 to 1 : 0.5,
and preferably additionally a surfactant or a surfactant mixture,
or
(a.3) an article according to any of the claims 7 to 9, preferably containing a surfactant or a surfactant mixture,
and
(b) applying or incorporating the fragrance and/or flavoring composition (a.1) or the compound / fragrances (a.2) or the article (a.3) to the hair or skin or fibers or surface to be perfumed, or into the room air to be perfumed in an amount sufficient for the consumer to perceive one or more scent and/or flavor notes selected from the group consisting of the notes green, herbaceous, fresh, fruity, floral, woody, animalistic, sweet, earthy, ambery, fatty, metallic, balsamic, leather note and chlorine note, preferably at least one of the scent and/or flavor notes selected from the group consisting of the notes green, woody, animalistic, earthy, ambery, fruity, floral, leather note and chlorine note.

## Revendications

1. Utilisation du composé de formule (I), 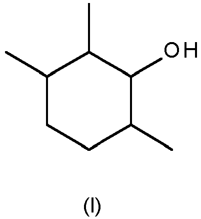 en tant que substance odoriférante et/ou aromatisante pour conférer, modifier et/ou intensifier une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence une ou plusieurs des notes olfactives et/ou gustatives choisies dans le groupe constitué par les notes verte, boisée, animale, terreuse, ambrée, fruitée, fleurie, la note de cuir et la note de chlore.

2. Composition de substance odoriférante et/ou aromatisante comprenant ou se composant du composé de formule (I) 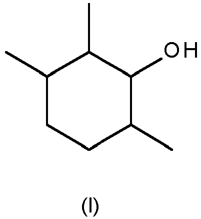
et d'une, de deux, trois, quatre, cinq, six, sept, huit, neuf, dix ou plusieurs autre(s) substance(s) odoriférante(s) et/ou aromatisante(s), dans lequel le rapport pondéral de la quantité totale de composé de formule (I) à la quantité totale d'autre(s) substance(s) odoriférante(s) et/ou aromatisante(s) se situe dans la plage allant de 1 : 1000 à 1 : 0,1, de préférence de 1 : 1000 à 1 : 0,5, et
dans laquelle la quantité totale de composé de formule (I) est contenue dans la composition de substance odoriférante et/ou aromatisante en une quantité qui est suffisante pour conférer et/ou intensifier une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence au moins l'une des notes olfactives et/ou gustatives choisies dans le groupe constitué par les notes verte, boisée, animale, terreuse, ambrée, fruitée, fleurie, la note de cuir et la note de chlore, et/ou pour modifier une ou plusieurs notes olfactives et/ou gustatives d'une ou bien de la/des autres substances odoriférantes et/ou aromatisantes de la composition de substance odoriférante et/ou aromatisante en direction d'une ou de plusieurs de ces notes olfactives et/ou gustatives.

3. Composition de substance odoriférante et/ou aromatisante selon la revendication 2, dans laquelle la quantité totale de composé de formule (I) par rapport au poids total de la composition de substance odoriférante et/ou aromatisante, se situe dans la plage allant de 0,0001 à 90 % en poids.

4. Composition de substance odoriférante et/ou aromatisante selon l'une quelconque des revendications 2 ou 3, dans laquelle
(i) la ou bien une, plusieurs ou l'ensemble des substances odoriférantes et/ou aromatisantes confèrent, modifient et/ou intensifient (également) une ou plusieurs notes olfactives et/ou gustatives choisies dans le groupe constitué par les notes verte, herbacée, fraîche, fruitée, fleuries, boisée, animale, douce, terreuse, ambrée, graisseuse, métallique, balsamique, la note de cuir et la note de chlore, de préférence une ou plusieurs des notes olfactives et/ou gustatives choisies dans le groupe constitué par les notes verte, boisée, animale, terreuse, ambrée, fruitée, fleurie, la note de cuir et la note de chlore, de manière particulièrement préférée choisies dans le groupe constitué par les notes fruitée, fleurie et la note de chlore,
ou dans laquelle
(ii) ladite ou bien une, plusieurs ou toutes les autres substances odoriférantes et/ou aromatisantes confèrent, modifient et/ou intensifient une ou plusieurs autres notes olfactives et/ou gustatives que celles mentionnées dans (i).

5. Utilisation d'une composition de substance odoriférante et/ou aromatisante selon l'une quelconque des revendications 2 à **4,** pour conférer, modifier et/ou intensifier une ou plusieurs notes olfactives et/ou gustatives choisies dans le groupe constitué par les notes verte, herbacée, fraîche, fruitée, fleuries, boisée, animale, douce, terreuse, ambrée, graisseuse, métallique, balsamique, la note de cuir et la note de chlore, de préférence une ou plusieurs des notes olfactives et/ou gustatives choisies dans le groupe constitué par les notes verte, boisée, animale, terreuse, ambrée, fruitée, fleurie, la note de cuir et la note de chlore.

6. Procédé pour conférer, intensifier et/ou modifier une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence pour conférer, intensifier et/ou modifier une ou plusieurs notes olfactives et/ou gustatives choisies dans le groupe constitué par les notes verte, boisée, animale, terreuse, ambrée, fruitée, fleurie, la note de cuir et la note de chlore, comprenant ou constitué par les étapes suivantes consistant à:
(a) fournir
(a.1) le composé de formule (I) 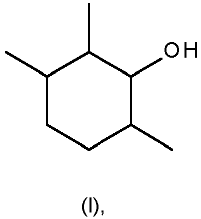 et
(a.2) une, deux, trois, quatre, cinq, six, sept, huit, neuf, dix ou plusieurs autres substance(s) odoriférante(s) et/ou aromatisante(s) avec une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence avec une ou plusieurs notes olfactives et/ou gustatives choisies dans le groupe constitué par les notes verte, boisée, animale, terreuse, ambrée, fruitée, fleurie, la note de cuir et la note de chlore,
(b) ajouter le composé de formule (I) (a.1) à la ou aux autres substance(s) odoriférante(s) et/ou aromatisante(s) (a.2), en une quantité qui est suffisante pour conférer, intensifier et/ou modifier une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence en une quantité qui est suffisante pour conférer, modifier et/ou intensifier une ou plusieurs notes olfactives et/ou gustatives choisies dans le groupe constitué par les notes verte, boisée, animale, terreuse, ambrée, fruitée, fleurie, la note de cuir et la note de chlore.

7. Article parfumé et/ou aromatisé, comprenant ou se composant
(i) d'une composition de substance odoriférante et/ou aromatisante selon l'une quelconque des revendications 2 à 4,
ou
du composé de formule (I) 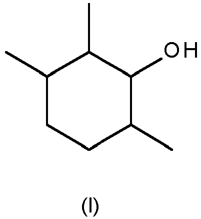 et d'une, de deux, trois, quatre, cinq, six, sept, huit, neuf, dix ou plusieurs autre(s) substance(s) odoriférante(s) et/ou aromatisante(s), dans lequel le rapport pondéral de la quantité totale de composé de formule (I) à la quantité totale d'autre(s) substance(s) odoriférante(s) et/ou aromatisante(s) se situe dans la plage allant de 1 : 1000 à 1 : 0,1, de préférence de 1 : 1000 à 1 : 0,5,
et
(ii) d'un ou de plusieurs autres composant(s), de préférence au moins un ou plusieurs, de préférence d'un, de deux, trois, quatre, cinq ou plus, additif(s), excipient(s) et/ou agent(s) actif(s), et
dans lequel ledit article est choisi dans le groupe constitué par les agents de lavage et les agents de nettoyage, les produits d'hygiène ou de soin, de préférence dans le domaine des soins du corps, des cheveux et de la bouche, de la cosmétique et du ménage, de préférence dans le groupe constitué par les nettoyants de surface, les nettoyants pour toilettes, les nettoyants tout usage, les bains de bouche, les dentifrices, les gels dentaires, les pâtes dentifrices, les poudres dentifrices, les chewing-gums dentaires, les extraits de parfum, les eaux de parfum, les eaux de toilettes, les eaux après-rasage, les Eaux de Cologne, les produits de pré-rasage, les Splash Cologne, les lingettes rafraîchissantes parfumées, les agents de nettoyage acides, alcalins et neutres, les produits à rafraîchir les textiles, les aides au repassage, les détergents liquides, les détergents en poudre, les produits de prétraitement de linge, les adousissants de linge, les savons à laver, les pastilles lave-linge, les désinfectants, les désinfectants de surface, les assainisseurs d'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soins des cheveux, les désodorisants, les antiperspirants, les produits de la cosmétique décorative, les bougies, les huiles de lampe, les bâtonnets d'encens, les insecticides, les répulsifs et les carburants.

8. Article parfumé et/ou aromatisé selon la revendication 7, dans lequel le composant (i) est contenu en une quantité qui est suffisante pour qu'un consommateur perçoive une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence une ou plusieurs notes olfactives et/ou gustatives choisies dans le groupe constitué par les notes verte, boisée, animale, terreuse, ambrée, fruitée, fleurie, la note de cuir et la note de chlore.

9. Article parfumé et/ou aromatisé selon l'une quelconque des revendications 7 ou 8, dans lequel la quantité totale de composé de formule (I), par rapport au poids total de l'article se situe dans la plage allant de 0,00001 à 10 % en poids, de préférence de 0,0001 à 5 % en poids, de manière particulièrement préférée de 0,001 à 2 % en poids, en outre de préférence de 0,005 à 1 % en poids.

10. Procédé destiné à parfumer et/ou aromatiser un article, comprenant ou se composant des étapes suivantes consistant à:
(a) fournir
(a.1) une composition de substance odoriférante et/ou aromatisante selon l'une quelconque des revendications 2 à 4,
ou
(a.2) le composé de formule (I), 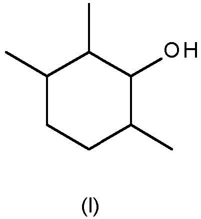 et en option une, deux, trois, quatre, cinq, six, sept, huit, neuf, dix ou plusieurs autre(s) substance(s) odoriférante(s) et/ou aromatisante(s), dans lequel le rapport pondéral de la quantité totale de composé de formule (I) à la quantité totale d'autre(s) substance(s) odoriférante(s) et/ou aromatisante(s) se situe dans la plage allant de 1 : 1000 à 1 : 0,1, de préférence de 1 : 1000 à 1 : 0,5,
et
(b) ajouter la composition de substance odoriférante et/ou aromatisante (a.1) ou bien le composé / les substances odoriférantes (a.2) à l'article à parfumer et/ou aromatiser, en une quantité qui est suffisante pour conférer, intensifier et/ou modifier une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence une ou plusieurs des notes olfactives et/ou gustatives choisies dans le groupe constitué par les notes verte, boisée, animale, terreuse, ambrée, fruitée, fleurie, la note de cuir et la note de chlore.

11. Procédé destiné à parfumer les cheveux, la peau, des fibres textiles, des surfaces et/ou de l'air ambiant, comprenant ou se composant des étapes suivantes consistant à:
(a) fournir
(a.1) une composition de substance odoriférante et/ou aromatisante selon l'une quelconque des revendications 2 à 4, contenant de préférence un tensioactif ou un mélange de tensioactifs,
ou
(a.2) le composé de formule (I), 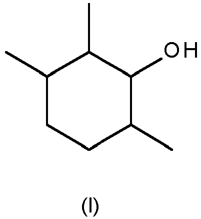
et en option une, deux, trois, quatre, cinq, six, sept, huit, neuf, dix ou plusieurs autre(s) substance(s) odoriférante(s) et/ou aromatisante(s), dans lequel le rapport pondéral de la quantité totale de composé de formule (I) à la quantité totale d'autre(s) substance(s) odoriférante(s) et/ou aromatisante(s) se situe dans la plage allant de 1 : 1000 à 1 : 0,1, de préférence de 1 : 1000 à 1 : 0,5,
et, en outre, de préférence un tensioactif ou un mélange de tensioactifs,
ou
(a.3) un article selon l'une quelconque des revendications 7 à 9, de préférence contenant un tensioactif ou un mélange de tensioactifs,
et
(b) appliquer ou bien introduire la composition de substance odoriférante et/ou aromatisante (a.1) ou bien le composé / les substances odoriférantes (a.2) ou bien l'article (a.3) sur les cheveux ou bien la peau ou bien les fibres ou bien la surface à parfumer ou dans l'air ambiant à parfumer en une quantité qui est suffisante pour que le consommateur perçoive une ou plusieurs notes olfactives et/ou gustatives choisies dans le groupe constitué par les notes verte, herbacée, fraîche, fruitée, fleurie, boisée, animale, douce, terreuse, ambrée, graisseuse, métallique, balsamique, la note de cuir et la note de chlore, de préférence au moins une des notes olfactives et/ou gustatives choisies dans le groupe constitué par les notes verte, boisée, animale, terreuse, ambrée, fruitée, fleurie, la note de cuir et la note de chlore.
